# EUROPEAN PATENT APPLICATION

(11) **EP 3 958 273 A1**
(43) Date of publication of application: **23.02.2022**
(21) Application number: 20191364.7
(22) Date of filing: 17.08.2020
(51) Int. Cl.: G16H 20/30, G16H 40/63, G16H 40/67

(54) **AN ORAL CARE SYSTEM FOR GRANTING ACCESS TO A NETWORK-BASED SERVICE**

(71) Applicant: Koninklijke Philips N.V., 5656 AG Eindhoven (NL)
(72) Inventor: GERHARDT, Lutz Christian, 5656 AE Eindhoven (NL); TE VELDE, Mart Kornelis-Jan, 5656 AE Eindhoven (NL); JOHNSON, Mark Thomas, 5656 AE Eindhoven (NL)
(74) Representative: Philips Intellectual Property & Standards

(57) **Abstract**

The present invention is directed to an oral care system configured for granting access to a network-based service, to a method of granting access for an oral care system to a network-based service, to a computer program, to a controller and to a code-carrying element comprising a code. In embodiments, the oral care system (100) is configured to allow an establishing of a data exchange (108) between the oral care system (100) and a computer network structure (300). The oral care system (100) comprises a data reader (102) for receiving a code (200), wherein the oral care system (100) is configured for generating a control signal (104) based on the received code (200). The oral care system (100) is also configured for sending the generated control signal (104) to the network (300) for initiating a generation of a server signal (106) in the computer network structure (300), which will be received by the oral care system (100). In this way, network-based services are accessed by the oral care system (100). In embodiments, the oral care system is configured for reconfiguring software comprised in the oral care system (100) thereby granting the access to the network-based service. The software reconfiguration carried out by the oral care system comprises unlocking software installed on the oral care system (100), installing new software on the oral care system (100), and/or changing software installed on the oral care system.

## Description

### FIELD OF THE INVENTION

The present invention is directed to an oral care system configured for granting access to a network-based service, a method of granting access for an oral care system to a network-based service, a computer program, a controller and a code-carrying element comprising a code.

### BACKGROUND OF THE INVENTION

Dental and oral health is an essential part of overall health and well-being. Poor oral hygiene can lead to dental cavities and gum disease, and has also been linked to heart disease, cancer, and diabetes.

Maintaining healthy teeth and gums is a lifelong commitment. The earlier one learns proper oral hygiene habits, such as brushing, flossing, and limiting your sugar intake, the easier it will be to avoid costly dental procedures and long-term health issues.

Moreover, in the field of oral care systems using toothbrushes and flossing devices for oral cavities, interconnected devices become more and more important. However, the inventors of the present invention found that these connected systems are often difficult to handle for a user of the oral care system without specific knowledge.

### SUMMARY OF THE INVENTION

It may be seen as an object of the present invention to provide for an improved oral care system.

The present invention is defined by the independent claims. Further embodiments and advantages of the present invention are incorporated in the dependent claims and the description.

The described embodiments similarly pertain to the oral care system configured for granting access to a network-based service, to the method of granting access for an oral care system to a network-based service, to the computer program, to the controller and to the code-carrying element comprising a code. Synergetic effects may arise from different combinations of the embodiments although they might not be described in detail.

Further on, it shall be noted that all embodiments of the present invention concerning a method, might be carried out with the order of the steps as described, nevertheless this has not to be the only and essential order of the steps of the method. The herein presented methods can be carried out with another order of the disclosed steps without departing from the respective method embodiment, unless explicitly mentioned to the contrary hereinafter.

Technical terms are used hereinafter in their common sense. If a specific meaning is conveyed to certain terms, a definition of terms will be given in the following in which the terms are used.

According to a first aspect of the present invention, an oral care system for granting access to a network-based service is presented. The oral care system comprises a data reader for reading a code comprised in a code-carrying element, wherein the oral care system is configured to send a signal based on the code read by the data reader to a receiver of a network outside the oral care system. Further, the oral care system is configured for granting access to the network-based service in response to sending the signal to the receiver of the network. Using a code to get access to a service may remove the need for a user to, for instance, go to an online portal to register in order to get access to such a service.

In other words, the oral care system can read said code by the data reader and can subsequently send a signal to the external network, which in turn triggers that the oral care system, and thus the user of said system, gets access to at least one network-based service that is used, processed, displayed and/or applied by the oral care system. The oral care system may e.g. itself unlock or activate the connectability to said network thereby granting said access. This may be carried out by using e.g. WiFi or the internet to connect with said network. However, said unlocking or activating may alternatively only be carried out after the oral care system has received a server signal from said network. Both alternative embodiments will be explained in more detail hereinafter. Thus, as will be appreciated by the skilled reader, in another aspect of the present invention, the entire system comprising at least the oral care system and said network is provided. Also this will be explained in more detail hereinafter.

One advantage of the oral care system and the corresponding method of the present invention is that by replacing e.g. a cleaning head by a new cleaning head comprising said code, a functionality of the oral care system and/or the network can be unlocked. This can lead to new business models, which can generate added value for a user of the oral care system. A further advantage of this system and method is that further functionalities and/or analysis can be performed on the oral care system and/or in the network (e.g. a data analysis of usage data measured by the oral care system), which can lead to an improved oral care behavior of the user, and/or longer operating time of the oral care system, because less computing power is needed within the oral care system. This will become apparent from the following detailed explanation.

In the context of the present invention, the network can e.g. be a cloud system like a cloud computing network and/or a cloud storing database. It should be noted that in the context of the present invention the term "network" is used synonymously with the term "computer network structure".

Moreover, in the context of the present invention, the term "network-based service" may be understood as a network-based functionality that can be sued by the user of the oral care system. As will become apparent from the following disclosure, the technical functionality of the oral care system is supplemented and/or changed due to the process of reading out the code, sending the signal to the network and granting access to said network-based service. Thus, the term "network-based service" is synonymously used with the term "network-based functionality".

Further, the term "data exchange" shall be understood broadly in the context of the present invention. The data exchange can be configured for uploading from the oral care system to the network and/or downloading from the network to the oral care system at least one data element, e.g. in a constant manner and/or at a predefined rate or at a variable rate. In other words, the oral care system can be configured for, constantly and/or at a predefined rate or a variable rate, uploading to the network and/or downloading from the network at least one data element due to the software reconfiguration. The data element can be any type of signal or program code, which can be processed and/or executed within the oral care system.

In an exemplary scenario of the present invention, the user of such an oral care system wants to upgrade his system with an additional diagnostic functionality, like e.g. a diagnostic analysis of oral care data measured by his/her system. Such an analysis is carried out in this embodiment e.g. on a server of the applicant of the present application. In such a scenario, the user may buy as a code-carrying element a replaceable brush head, which is to be received by the long lasting handle of the oral care system. Another example is the replacement of an old handle of the oral care system by a new handle (being the code-carrying element) comprising code that can be read out by e.g. a long lasting base station of the oral care system. However, in the following the example with the code-carrying element being a replaceable brush head will be elucidated in more detail without limiting the present invention to this example.

The replaceable brush head may be available for the user in several different variants, each variant storing different code, which different code in turn causes the grant of access to different network-based service. When said code is being read out by the data reader comprised in the oral care system (e.g. by the reusable handle of the oral care system), it is triggered that a sender of the oral care system starts to communicate with the external network outside the oral care system. The oral care system in this way can receive from and/or exchange data with said network, which allows offering the user network-based services, which he/she did not have in his oral care system before said replaceable brush head was put together with said handle.

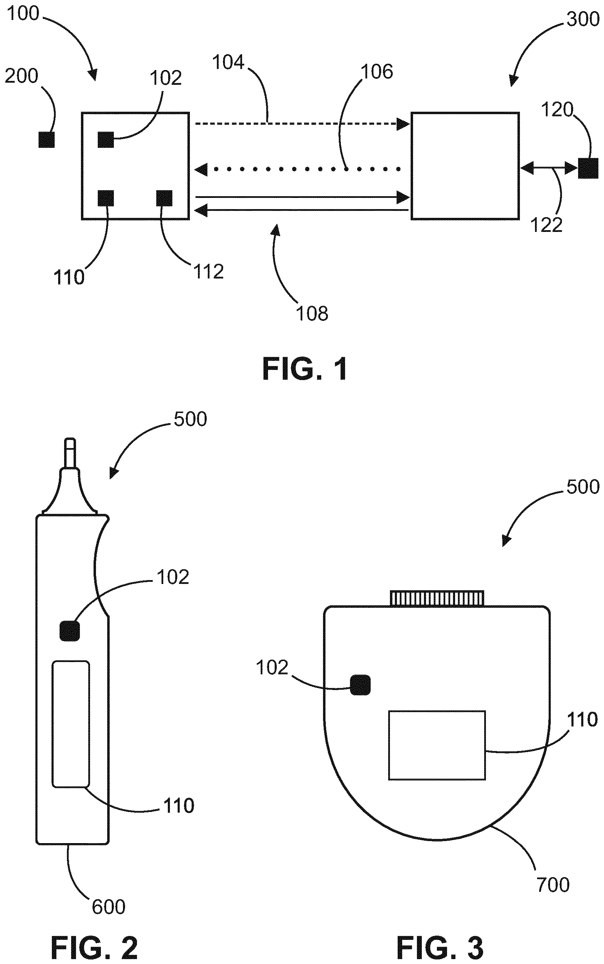

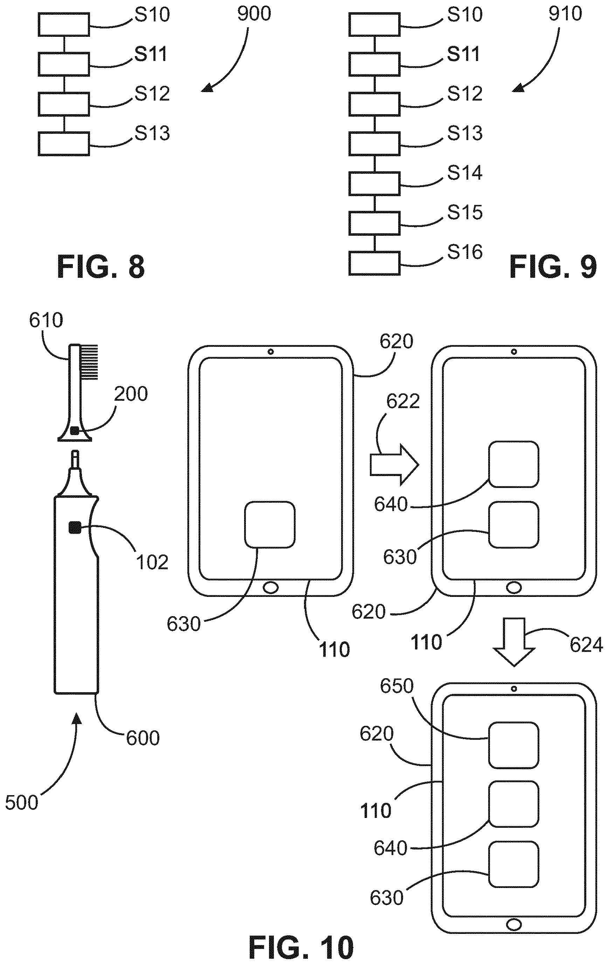

As will be explained in detail hereinafter in the context of particular embodiments, the oral care system may, upon reading said code, run a software reconfiguration such that the user and the oral care system get access to said network-based service. In such embodiments, sending the signal/code by the oral care system to the network may initiate said software reconfiguration. For example, in response to reading the code and sending the signal by the oral care system the network can be requested by the oral care system to send software to the system or the system/the network can unlock software that is already installed on the oral care system. Such procedures of sending and installing software or of unlocking software may be caused in response to the oral care system sending the signal based on the code. Detailed embodiments thereof are explained hereinafter, e.g. in the context of figures 1 and 10.

Generally, the signal that is sent to the network by the oral care system may just be the code, which was read out by the data reader. However, as is understood by the skilled reader, in other embodiments, the signal sent to the network is associated with said code or reflects said code in that sense that the code codifies access to a particular network-based service. The sent signal causes, on either the oral care system side, or on the network side, or on both sides that the oral care system gets access to said codified network-based services.

As was mentioned before, the oral care system comprises a data reader. The term "data reader" shall, in the context of the present invention, be understood broadly. The data reader can be any device configured for reading out a code, in particular from a data storage/code-carrying element. For example, the data reader can be a QR code reader or an RFID reader or may be part of an wire-bound or wireless plug-and-socket system for transferring the code from the code-carrying element to the oral care system.

Further, the "code" can be any kind or form of data, token or identifier, which can store specific information and/or commands, which when being read cause the oral care system to initiate the process of granting access to code-specific network-based service. Moreover, the oral care system can, as a consequence of reading out the code, cause that the signal is sent to the receiver of the network.

Accordingly, the oral care system can comprise a controller or a computing logic, which can be configured for generating, calculating and/or determining the signal to be sent to the network based on the code that was read out. Note that the term "controller" shall be understood broadly, in particular as any form of computing or processing device and shall comprise any calculation unit/processor and comprise also a programmable controller.

Furthermore, the oral care system can be configured for sending, transmitting and/or dispatching the generated signal to the network via a sender within the oral care system. The signal can be configured for initiating, triggering and/or starting a generation of a server signal, i.e. a network signal, in the network. In other words, the generated signal, e.g. a command in form of a program code for initiating the generation of the server signal in the network, is sent from the oral care system to the network, which receives and processes the signal.

Furthermore, the oral care system, e.g. a controller of system, is configured for receiving, downloading and/or obtaining a server signal generated by the network. Moreover, the oral care system, e.g. the controller of the system, is configured for processing, computing and/or converting the received server signal generated by the network.

It should be noted an oral care system of the present invention may comprise several different components. For example, a base station may be comprised, and/or a handle for receiving a cleaning head being e.g. a brush head and/or a flossing head may be comprised, and/or a cleaning head to be received by a handle of the oral care system may be comprised, wherein the cleaning head is a brush head and/or a flossing head, and/or a mouthpiece configured for brushing teeth, flossing teeth and/or soft tissue treatment may be comprised, and/or a user interface may be comprised (which may be embodied as e.g. a smart phone, a tablet, a computer, or as an interface integrated into a cleaning appliance of the oral care system), and/or a code-carrying element may be comprised (like e.g. a scratch card and/or a packaging for a cleaning head of an oral care system). This will be elucidated in more detail in the context of particular embodiments.

In the following an example of the present invention will be explained. In this exemplary embodiment, the oral care system comprises a handle of an electric toothbrush and a mobile device, like a tablet or a smartphone. The handle can be wirelessly connected to the mobile device, e.g. via Bluetooth. The handle of the oral care system comprises the data reader, which can read-out the code from a brush head, which can be attached to the handle. The handle can transfer the read code to the mobile device, e.g. via Bluetooth. The mobile device, which is explicit part of this oral care system, can then generate a signal based on the read code, which specifies at least the network based service to be granted access to. In another embodiment the code also specifies the user of the oral care system and/or the type of data exchange which can be caused between the oral care system and the network.

In this embodiment, the oral care system can send, e.g. via the mobile device, the generated signal to a network or a computer network structure for example a cloud system, e.g. via an internet or using a Wi-Fi connection established due to instructions contained in the code that was read-out. The network/cloud system can send a server signal to the oral care system as an answer to said signal. The server signal can further specify which kind or type of data exchanges can be caused between the oral care system and the cloud system in order to provide the user with said network-based service.

For example, the code can comprise a command to activate a gum-line surveillance mode in the oral care system. Based on the code the signal is generated, which comprises the command to activate a gum-line surveillance mode and user specifications of the user of the oral care system. Based on said signal the cloud system can then generate a server signal, which comprises a command for initiating a software reconfiguration by updating the software installed on the oral care system accordingly. Thereby the server signal can be specifically generated for a single, individual oral care system according to the signal, which was generated dependent on the individual code of the brush head that was read-out by the oral care system.

The oral care system, in particular the mobile device comprised by the oral care system, can receive and process this server signal. Furthermore, the oral care system can start a software reconfiguration of a software, which was already installed on e.g. the mobile device and/or on the oral care handle, or which was transferred to the oral care system based on instructions contained in said code. After this software reconfiguration the oral care system can provide/generate a particular data exchange between the oral care system and the network. The data exchange can be used for enabling further functionalities of the oral care system and/or the network.

Thus, by simply attaching the brush head, which stores the code, onto the handle, the oral care system can establish a data exchange between the oral care system and the cloud system for unlocking and/or activating network-based services, i.e. further functionalities, which may use the established data exchange. This will be described in more detail in the context of the embodiment shown in e.g. figures 1 and 10.

According to an exemplary embodiment of the present invention, the oral care system is configured for reconfiguring software comprised in the oral care system thereby granting the access to the network-based service. The software reconfiguration carried out by the oral care system comprises unlocking software installed on the oral care system, installing new software on the oral care system, and/or changing software installed on the oral care system.

Such a software reconfiguration can thus comprise downloading data, e.g. a software update package, from the network and/or the internet. The software reconfiguration can be configured for reconfiguring and/or updating the software installed on the oral care system. Furthermore, the software reconfiguration can be configured for changing and/or adapting the software already installed on the oral care system based on an instruction comprised in the read code or e.g. in the received server signal, as will be explained hereinafter.

The oral care system may be configured in one embodiment for sending the signal to the network for initiating a generation of a server signal in the network, wherein the oral care system is configured for receiving and processing the server signal from the network. The oral care system is configured for initiating a software reconfiguration of a software installed on the oral care system based on the received server signal. The software reconfiguration of the software installed on the oral care system may allow for or may cause a data exchange, in addition to the signal sent to the network and in addition to the server signal, between the oral care system and the network, which can be used for providing the network-based service/functionality to the user via the oral care system.

There can be one or more software and/or software programs installed on the oral care system, in particular on a controller of the oral care systems. The installed software can be configured for operating and/or controlling at least one device and/or sensor and/or functionality of the oral care system.

Moreover, the oral care system, in particular the controller of the oral care system, can be configured for initiating, starting and/or triggering a software reconfiguration and/or software update of the software installed on the oral care system, in particular on the controller of the oral care system. Such a software reconfiguration may then supplement or add a functionality of any of said devices and/or sensors and/or functionalities of the oral care system. Exemplary embodiments thereof have been explained already and will be explained hereinafter in more detail.

According to an exemplary embodiment of the present invention, the oral care system comprises a user interface configured for using the network-based service.

Such a user interface can be provided as a separate, independent component of the oral care system, like e.g. a tablet screen that is wirelessly connected to e.g. a base station of the oral care system. However, the user interface may also be provided as an integral part of e.g. a base station or of a handle of the oral care system. The user interface may allow a visual, audio and/or information or data exchange between the user and the oral care system.

Said user interface is configured for using the network-based service. Thus, the user of the oral care system can make use of the network-based services via the user interface. For example, the user of such an oral care system wants to upgrade his system with an additional diagnostic functionality, like e.g. a diagnostic analysis of oral care data measured by his/her oral care system. The user for that purpose buys a new brush head comprising corresponding code, which when being read out unlocks software that was already stored in the oral care system and which performs said diagnostic analysis of the user's oral care data, like e.g. the frequency and duration of an average tooth brushing duration or typical movements of the user during such a procedure. In this example, the user interface is configured to share with the user in a visual, audio and/or haptic manner the results of the diagnostic analysis of such user data. It may also be the case that the user interface gets a new configuration based on the read out code to share said information. Such a new configuration may be unlocked/installed on the user interface by said software reconfiguration. Also this will be explained in more detail hereinafter in the context of particular embodiments.

In an embodiment, the user interface is embodied as a smart phone, a tablet, a computer, and/or as an interface integrated into a cleaning appliance of the oral care system.

Examples of such user interfaces have been mentioned before and will be explained in more detail in the context of other embodiments, in particular in the context of the figures.

According to an exemplary embodiment of the present invention, the oral care system is configured to change a functionality of the user interface by carrying out the software reconfiguration.

In other words, the code can comprise the instruction that functionalities shall be installed in/on the user interface that were not yet present. Alternatively, the code can comprise the instruction to activate one or more technical features of the user interface that was already present, but not yet active, like e.g. 'greyed-out features' like greyed-out areas of a display being the user-interface. The changed functionality of the user interface may allow or facilitate the use of the network-based service to which the oral care system gets access after the code has been read.

According to an exemplary embodiment of the present invention, the oral care system is configured, after having granted access to the network-based service, to provide to the user a diagnostic functionality regarding oral health of the user.

Note that said diagnostic functionality is one example of a network-based service.

This embodiment comprises, but is not necessarily limited to, that said diagnostic functionality is carried out or provided by the oral care system itself. The diagnostic functionality may be provided completely or in part by the network and the oral care system receives the corresponding data from the network and presents the service/said received data/said diagnostic functionality to the user via e.g. the aforementioned user interface. In other words, any calculation of data may be carried out at the backend, i.e. within the network to which the oral care system is connected, and after the oral care system has received said data / calculation results, the user interface is used to provide such results to the user visually, in a haptic manner and/or acoustically.

In an example, the software reconfiguration causes or enables a remote diagnosis procedure between the oral care system, in particular the user interface of the oral care system, and the network by means of a data exchange that is generated upon reading out said code. In this example, said remote diagnosis procedure is the network-based service to which the oral care system gets access upon reading the code. In an example, such a remote diagnosis procedure causes access for the oral care system to a communication device like a computer or server of a dental professional. One advantage of this embodiment is diagnostic functionality that with the help of the remote diagnosis procedure medical suffering can be detected much faster since qualified personnel can access the oral care system with the data exchange.

The granting said access to the oral care system and/or a software reconfiguration can cause, enable and/or activate a remote diagnosis procedure. The remote diagnosis procedure can be caused between the oral care system, in particular the user interface of the oral care system, e.g. a tablet screen, and the network, e.g. a cloud system by means of the data exchange that is provided after the code has been read from the code-carrying element. Thus, in a particular embodiment the software reconfiguration can establish a data exchange between the oral care system and the network and can enable the remote diagnosis procedure between the oral care system and the network.

As was explained hereinbefore, the remote diagnosis procedure can be any form of procedure and/or instructions related to the oral care system and/or an oral care procedure carried out by said oral care system. For example, the remote diagnosis procedure allows a contact/communication, e.g. by a video connection, between a user of the oral care system and a dental professional, who has access to the network. Thereby the user of the oral care system can interact with the dental professional, in particular can ask questions/send data or the like, by means of the user interface of the oral care system and the user can receive answers to said question/data from the dental professional/network by the data exchange.

All these advantages can be provided to the user e.g. by said exemplary and non-limiting example of a brush head comprising code to activate the remote diagnosis procedure when being attached to the handle of the oral care system, thereby causing the software reconfiguration based on the command of the brush head. The same holds true for any kind of cleaning head to be received by a handle of an oral care system, a handle of an oral care system, a mouthpiece of an oral care system configured for brushing teeth, flossing teeth and/or soft tissue treatment, a scratch card, and/or a packaging for a cleaning head of an oral care system, as they are all exemplary embodiments of code-carrying elements.

According to an exemplary embodiment of the present invention, the oral care system is configured for uploading oral health data of the user of the oral care system to the network, and wherein the diagnostic functionality provided by the oral care system is based on the oral health data uploaded to the network.

In this embodiment, the software reconfiguration may cause or allow for an upload of individual use data of the oral care system and causes or allows for a download, from the network, of individual user instructions that are generated by the network based on the uploaded individual use data. In particular, the user interface of the oral care system may be configured for carrying out such an upload and/or download.

One advantage of this embodiment is that with the help of the individual use data of the oral care system in combination with the data exchange, individual user instructions can be provided in detail and in a constant manner, e.g. via the user interface, since the beneficial computing power of the network and dental knowledge that may be stored in the network can be beneficially used for generating the user instructions.

The software reconfiguration, described hereinbefore and hereinafter, can cause, trigger and/or activate an upload/sending of individual use data of the oral care system to the network by means of the data exchange. Therefore, use data of the oral care system, e.g. sensor data regarding e.g. brushing teeth by the user, can be uploaded to the network by means of the data exchange after the software reconfiguration is completed on the oral care system. Further, the software reconfiguration can cause, trigger and/or activate downloading/receiving individual user instruction by the oral care system, which are generated by the network based on the individual use data that were uploaded by the oral care system by means of the data exchange. Such upload and download functionalities of the oral care system are understood by the skilled reader as an example of the network-based service to which access is granted when the oral care system reads the corresponding code.

In other words, the oral care system can download/receive from the network by means of the data exchange individual user instructions, which can in particular be provided to the user by means of the user interface of the oral care system, which were generated based on the uploaded individual use data by the network. For example, the oral care system uploads after the software reconfiguration brushing habits, in the form of measurement data, of a user of the oral care system to the network, when a brush head storing code is attached to the oral care system, said code comprising a command for triggering the software reconfiguration. Thereby the oral care system can upload the brushing habits of said user and can download individual user instructions, e.g. changing a brushing angle and/or highlighting particular areas of the oral cavity that need to be cleaned more properly. This addresses said brushing habits in order to improve the brushing result of an oral care procedure performed by the oral care system.

According to an embodiment, the oral care system is configured for outputting the downloaded individual user instructions to a user of the oral care system via the user interface of the oral care system.

One advantage of this embodiment is that with the help of the output of the downloaded individual user instructions to the user oral care habits of said user can beneficially be improved.

The term "output" shall be broadly understood and comprises at least any form of visual, acoustic and/or haptic output. For example, the oral care system comprises a tablet with a screen and speakers, and said tablet can output the downloaded individual user instructions in form of images, videos and acoustic signals by means of the screen and speakers of said tablet to the user.

According to an exemplary embodiment of the present invention, the oral care system is configured, after having granted access to the network-based service, to provide to the user an insurance functionality.

For this purpose, a data connection between an external database and the network can be established based on the signal sent by the oral care system to the network. Thus, in this embodiment the external data base is not part of the network.

The external data base may be used by an insurance and further data like statistical oral care data may be stored in said external database. In that sense, the oral care system is configured to generate a signal, which triggers such a data connection after having read said code. Moreover, the oral care system is configured to send oral care data, e.g. measurement data of a sensor measuring during an oral care procedure, to the network and is configured to cause the network to exchange the oral care data received from the oral care system with the external database via the established data connection. This data can be stored in the external database and used for further analysis. One advantage of this embodiment is that data generated by the oral care system and/or the network can be compared to other data sets in order to further improve the oral care procedure performed by the oral care system and the network.

According to an embodiment, the oral care system is configured for granting access to an external database in response to sending the signal to a receiver of the network, wherein the oral care system is configured to exchange oral care data generated/measured with the oral care system between the external database and the oral care system. This data exchange with the external data base is an embodiment of the network-based service to which access is granted upon reading said code.

One advantage of this embodiment is that data generated by the oral care system can be compared to other data sets in the external database in order to further improve the oral care procedure performed by the oral care system. Moreover, third parties, e.g. insurances and the like, can access the oral care data stored in the external database. In other words, the oral care system can connect to an external database and can input into said external database data, which were generated/measured by the oral care system. The data generated by the oral care system can be for example measurement values of a kind of a sensor used during an oral care procedure of the oral care system. This data can be stored in the external database and used for further analysis. In particular, a third party, for example an insurance provider can access the generated data and can perform further analysis on said data. More details can gathered from figure 9, in which also such insurance functionalities are described.

According to an exemplary embodiment of the present invention, the oral care system is configured for uploading, as health data of the user, data based on at least one image of tissue in an oral cavity of the user.

In other words, the oral care system is configured for transmitting the generated at least one image of the oral cavity to the network by means of e.g. the data exchange as was explained hereinbefore.

One advantage of this embodiment is that with the help of such an image in combination with the computing power of the network e.g. a complex image recognition/analyzing algorithm can be performed on images of the user's oral cavity. This enables new forms of dental care, because the image data can be processed much more accurate and the results of a such backend calculation can be provided to the oral care system.

According to an exemplary embodiment, the oral care system comprises a camera configured for generating said image of the user's oral cavity.

Said camera may be located in a brush head, handle or mobile device of the oral care system. The camera is configured for generating, capturing and/or taking images of an oral cavity of the user of the oral care system. In other words, the camera can take images of teeth and gum within an oral cavity of the oral care system's user. Furthermore, the oral care system can be configured for transmitting, uploading and/or sending the generated images to the network by means of the data exchange after the code has been read out and after the signal has been sent to a receiver of the network and/or after the corresponding software reconfiguration. Furthermore said software reconfiguration can cause to transmit, upload and/or send the generated at least one image of the oral cavity to the network by means of the data exchange.

For example, the camera is located in a brush head of the oral care system. In this example the brush head also comprises the code, which stores the command to trigger the software reconfiguration such that the oral care system is provided with the upload functionality for uploading the one or more images taken by the camera of the brush head to the network. The network can beneficially use these images e.g. for improving individual user instructions.

According to an exemplary embodiment of the present invention, the oral care system is configured, in order to provide the diagnostic functionality, to receive from the network data about a second opinion regarding the oral health of the user, to process said received data about the second opinion, and to share the data about the second opinion with the user via a user interface of the oral care system.

According to an exemplary embodiment of the present invention, the oral care system comprises an oral care appliance, and the oral care system is configured to upload to the network usage data indicative for a use of the oral care appliance, and the oral care system is configured to receive and process usage feedback data from the network as network-based service.

The comprised oral care appliance may e.g. brushing teeth, flossing teeth, soft tissue treatment of tissue in the oral cavity, and any combination thereof.

The oral care system may comprise one or more sensors for measuring the oral care habits of the user with regard to one or more of said oral care appliances. Said one or more sensors may thus measure the usage data indicative for a use of the oral care appliance.

It should be noted that the term "process usage feedback data" is to be understood in its broadest possible sense. In other words, in one embodiment the oral care system is configured upon receipt of said usage feedback data to simply share said data with the user via e.g. the user interface. However, in another embodiment the user interface carries out a calculation based on the received feedback data and displays the corresponding calculation results to the user. Other embodiments of processing such usage feedback data are comprised as well.

According to a second aspect of the present invention, a system comprising at least the oral care system and said network is provided.

In exemplary embodiments thereof, the network/computer network structure is a cloud system, e.g. a cloud computing network and/or a cloud storing database.

According to third aspect of the present invention, a method of granting access for an oral care system to a network-based service is presented. The method comprises the steps of reading, by a data reader of an oral care system, a code comprised in a code-carrying element, sending a signal, based on the code read by the data reader, by the oral care system to a receiver of a network outside the oral care system, and granting access to a network-based service in response to sending the signal to the receiver of the network.

The method can be performed on an oral care system in particular on a controller of the oral care system. Further, the steps of the method can be carried out in any sequence, which is understood by the skilled reader as technical reasonable.

The oral care system can read said code by the data reader and can subsequently send a signal to the external network, which in turn triggers that the oral care system, and thus the user of said system, gets access to at least one network-based service that is used, processed, displayed and/or applied by the oral care system. This method may be carried out entirely by the oral care system, but in other embodiments the method may be carried out in part by the oral care system and in part by the network to which the oral care system gets, at least temporarily, connected to after having read the code and after having sent said signal to the receiver of the network.

According to an exemplary embodiment of the present invention, the method also comprises presenting data relating to a diagnosis and/or usage feedback to the user.

The method of the present invention may comprise the following steps of receiving a code by a data reader of the oral care system, generating a control signal in the oral care system based on the received code, sending from the oral care system the generated control signal to a network thereby initiating a generation of a server signal in the network, receiving and processing by the oral care system the server signal from the network thereby granting said access to said network-based service.

In the following embodiments of this method are explained, which can all be combined with each other. According to an exemplary embodiment, this method comprises the step of initiating a software reconfiguration on the oral care system based on the received and processed server signal. In an even further embodiment, the method also comprises the step of causing due to the software reconfiguration a data exchange, in addition to the control signal and the server signal, between the oral care system and the network. The method can comprise the step of receiving, downloading and/or transmitting the code by/ by means of the data reader of the oral care system. Moreover, the step of generating, computing and/or determining a control signal in the oral care system based on the received code can be comprised by the method. The step of generating the control signal can comprise the step of receiving user specifications and of generating the control signal based on the user specifications and the received code. Moreover, the method can comprise the step of sending, uploading and/or transmitting from the oral care system the generated control signal to a network, e.g. by means of an internet connection. The sending of the generated control signal can cause, initiate and/or trigger a generation/computation of a server signal in the network.

The method can also comprise the step of receiving by the oral care system the server signal from the network, in particular a server signal which was generated by the network as an answer to the uploaded control signal. Moreover, the step of processing, computing and/or handling the server signal by the oral care system can be comprised by the method. Further, the method may comprise the step of initiating/triggering a software reconfiguration at the oral care system, in particular at a controller of the oral care system. According to an embodiment, the method comprises the step of uploading oral care data generated by the oral care system to the network by means of the data exchange. One advantage of this embodiment is that with the help of the upload computing power can be saved on the oral care system and therefore other tasks can be performed on the oral care system, since the evaluation of sensor data can be performed on the network.

According to an embodiment, the method comprises the step of causing a remote diagnosis procedure between the oral care system, in particular between a user interface of the oral care system, and the network by means of the data exchange. In this way, with the help of the remote diagnosis procedure the oral care of a user can be improved.

The method can also comprise the step of causing, activating and/or establish a remote diagnosis procedure between the oral care system, in particular the user interface thereof, and the network by means of the data exchange. In other words, the data exchange can provide a remote diagnosis procedure, e.g. an interaction between the user of the oral care system and a dental professional.

According to an embodiment, the method comprises the steps of uploading individual use data of the oral care system to the network, and downloading from the network structure user instructions that are generated by the network based on the uploaded individual use data. One advantage of this embodiment is that the efficiency of an oral care appliance/procedure, e.g. brushing teeth, can be improved by using said individual user instructions.

Furthermore, the method can comprise the step of uploading, sending and/or transmitting individual use data of the oral care system, in particular data about a user using the oral care system, to the network. Moreover, the step of downloading, receiving and/or requesting from the network individual user instructions that are generated/computed by the network based on the uploaded individual use data. In particular, the method for operating the oral care system can comprise the step of downloading by means of the data exchange from the network individual user instructions that are generated by the network based on the uploaded individual use data.

It should be noted that also a computer-implemented method for operating a computer network structure communicating with an oral care system is part of the present invention. Such a method comprises the steps receiving a signal from the oral care system by the network, selecting in the network at least one oral care functionality to which access is granted for the oral care system based on the received signal, generating a server signal based on the selected oral care functionality, wherein the server signal is configured for initiating a software reconfiguration of a software installed on the oral care system, and sending the generated server signal to the oral care system thereby initiating the software reconfiguration of the software installed on the oral care system.

In a particular embodiment, this method comprises the steps of establishing a data connection between an external database and the network based on the received control signal, receiving, by the computer network structure, oral care data generated by the oral care system, and exchanging the oral care data received from the oral care system between the external database and the network via the established data connection.

According to another fourth of the present invention, a computer program is presented, which instructs, when executed, a controller to control a system to perform a method of granting access for an oral care system to a network-based service, the method comprising reading, by a data reader of an oral care system, a code comprised in a code-carrying element, sending a signal, based on the code read by the data reader, by the oral care system to a receiver of a network outside the oral care system, and granting access to a network-based service in response to sending the signal to the receiver of the network.

The computer program may be part of a computer program, but it can also be an entire program by itself. For example, the computer program presented herein may be used to update an already existing computer program to get to the present invention.

The computer program may be stored on a computer readable medium such as for example, a USB stick, a CD, a DVD, a data storage device, a server, a hard disk, or any other medium on which a computer program as described above can be stored.

According to a fifth aspect of the present invention, a controller comprising a computer program as described herein is presented.

As was mentioned before, the term "controller" shall be understood broadly, in particular as any form of computing or processing device and shall comprise any calculation unit/processor and comprise also a programmable controller.

According to another aspect of the present invention, a code-carrying element comprising a code and configured to facilitate a read out of the comprised code by a data reader of an oral care system is presented. The comprised code is configured to allow granting access to a network-based service when sending a signal based on the code to a receiver of a network outside the oral care system.

Such code-carrying element may be e.g. a USB stick, a CD, a DVD, a data storage device, a server, a hard disk, or any other medium on which a code is stored, which is configured to allow granting access to a network-based service when sending a signal based on the code to a receiver of a network outside the oral care system. The code can be in a standard format for codes (e.g. a bar code). As was explained herein before, the code-carrying element comprises the code, which, when read by an oral care system, enables the system to get access to a network-based service.

According to an exemplary embodiment of the present invention, the code-carrying element is embodied as a cleaning head to be received by a handle of an oral care system, wherein the cleaning head is a brush head and/or a flossing head, a handle of an oral care system for receiving a cleaning head being a brush head and/or a flossing head, a mouthpiece of an oral care system configured for brushing teeth, flossing teeth and/or soft tissue treatment, a scratch card, and/or, a packaging for a cleaning head of an oral care system.

One advantage of this embodiment is that by replacing e.g. a cleaning head a functionality of the oral care system and/or the network can be unlocked. This can lead to new business models, which can generate added value for a user of the oral care system.

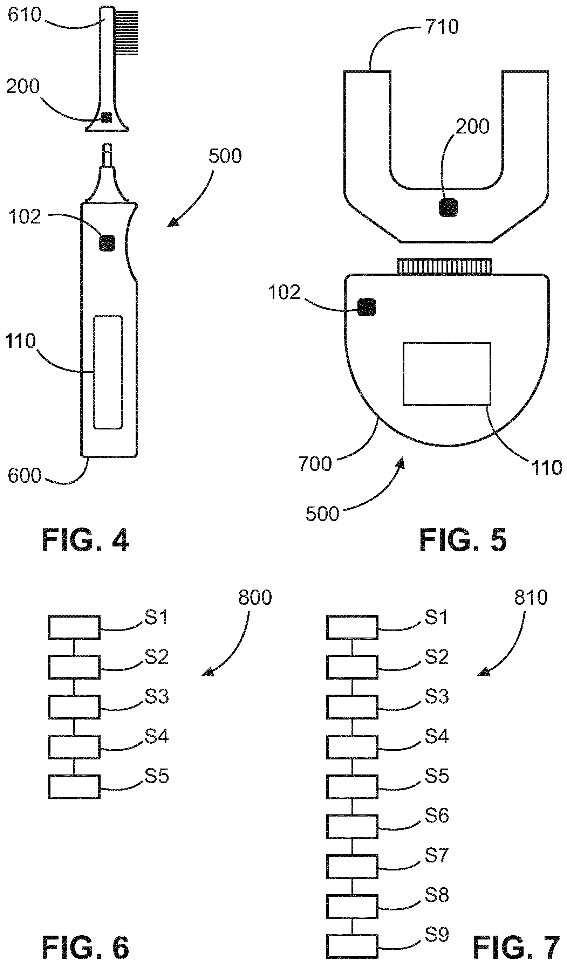

The replaceable cleaning part can be part of the oral care system. In particular, the replaceable cleaning part becomes a part of the oral care system when the replaceable cleaning part is attached to the support unit, e.g. to the handle or the mouthpiece. Furthermore, the replaceable cleaning part can comprise a data storage, for example a QR-tag on its outer surface and/or a RFID tag. The data storage of the replaceable cleaning part can store the code, which can be received by the support unit, the oral care system respectively. Thereby the software reconfiguration of the oral care system can be trigged by attaching the replaceable cleaning part to the handle or the mouthpiece, as will be explained in detail in view of figures 4 and 5 and their corresponding figure description.

Any feature or method step of an aspect of the present invention mentioned hereinbefore can feature or method step of another aspect.

These and other features of the invention will become apparent from and elucidated with reference to the embodiments described hereinafter.

The reference symbols used in the drawings, and their meanings, are listed in summary form in the list of reference symbols. In principle, identical parts are provided with the same reference symbols in the figures.

### BRIEF DESCRIPTION OF THE DRAWINGS

Figure 1 shows an oral care system and a network according to an embodiment of the present invention.
Figure 2 shows a handle of an oral care system according to an embodiment of the present invention.
Figure 3 shows a mouthpiece of an oral care system according to an embodiment of the invention.
Figure 4 shows a handle of an oral care system according to an embodiment of the invention.
Figure 5 shows a mouthpiece of an oral care system according to an embodiment of the invention.
Figure 6 shows a flow chart of a method according to an embodiment of the invention.
Figure 7 shows a flow chart of a method according to an embodiment of the present invention.
Figure 8 shows a flow chart of a method for operating a network according to an embodiment of the present invention.
Figure 9 shows a flow chart of a method for operating a network according to an embodiment of the present invention.
Figure 10 shows an oral care system according to an embodiment of the present invention.

### DETAILED DESCRIPTION OF EMBODIMENTS

Figure 1 shows an oral care system 100 configured for granting access to a network-based service. The oral care system 100 comprises a data reader 102 for reading a code 200 comprised in a code-carrying element. Such a code-carrying element can be embodied e.g. as a cleaning head to be received by a handle of the oral care system 100, wherein the cleaning head is a brush head and/or a flossing head. Alternatively, said code-carrying element could be a handle of the oral care system 100 for receiving a cleaning head, or a mouthpiece of the oral care system 100 configured for brushing teeth, flossing teeth and/or carrying out a soft tissue treatment, or a scratch card, and/or, a packaging of a cleaning head of an oral care system.

The oral care system 100 is configured to generate and send a signal 104 based on the code 200 read by the data reader 102 to a receiver of a network 300 outside the oral care system 100. The signal 104 may be in any embodiment mentioned herein a control signal. The oral care system 100 is configured for granting access to the network-based service in response to sending the signal to the receiver of the network 300. In other words, the oral care system 100 is configured to allow an establishing of a data exchange 108 between the oral care system 100 and the network 300. This can be understood as a basic configuration of an oral care system according to the present invention.

One advantage of this embodiment is that with the help of the data exchange 108 computing power resources within the oral care system 100 can be spared and/or used for other tasks, because the network 300 can be used with the help of the data exchange 108 for computing that needs to be done for the provision of the network-based service.

Additional functionalities of the oral care system will be described now with respect to the embodiment shown in figure 1, which are, however optional.

The oral care system 100 is configured for sending the generated signal 104 to the network 300 for initiating a generation of a server signal 106 in the network 300. Moreover, the oral care system 100 is configured for receiving and processing the server signal 106 received from the network 300. The oral care system 100 is configured for reconfiguring software comprised in the oral care system thereby granting the access to the network-based service.

The oral care system 100 can be configured for reconfiguring software comprised in the oral care system 100 thereby granting the access to the network-based service. Said software reconfiguration carried out by the oral care system can be embodied as unlocking software installed on the oral care system 100, installing new software on the oral care system 100, and/or changing software installed on the oral care system 100.

Furthermore, additional functionalities can be unlocked, installed and/or enabled within the oral care system 100 because the oral care system 100 can access more computing power and databases, which are stored in the network 300. Moreover, the oral care system 100 can provide access to further remote services via the established data exchange 108 between the oral care system 100 and the network 300.

The oral care system 100 comprises a camera 112 and also a user interface 110 is comprised. When the data reader 102 has read out the code 200 and the oral care system 100 has sent the signal 104 based on the read-out code 200 to the network 300, the network 300 establishes a video connection 122 or the like with a communication device 120 of a dental professional. This may allow a user of the system 100 to communicate with said dental professional. This communication is to be understood as an example of said network-based service to which the system 100 gets access upon reading said code 200.

Further, the network 300 can be configured for generating a server signal 108, which initiates the software reconfiguration, as was described hereinbefore in detail. Based on the software reconfiguration a data exchange 108 between the oral care system 100 and the network 300 is facilitated. In an exemplary embodiment, oral care data generated by the oral care system 100 are then transferred to the network 300 via the data exchange 108. The network can process the transferred oral care data and e.g. provide the data to the communication device 120 of the dental professional. The dental professional can enter instructions and/or hints into the communication device 120 for improving the oral care procedure performed by the oral care system 100. The network 300 can then provide these instructions to the oral care system 100 by means of the data exchange. The oral care system 100 can output these instructions by means of the user interface 110 of the oral care system 100 to the user of the oral care system 100. For example, a tablet of the oral care system comprises a screen and speakers, and said tablet can output these instructions in form of images, videos and acoustic signals by means of the screen and speakers of said tablet to the user.

Figure 2 shows a support unit 500 of an oral care system according to an exemplary embodiment of the present invention. The support unit is embodied as a reusable handle 600, which comprises the data reader 102 described hereinbefore. The handle 600 is configured to receive a cleaning head, wherein the cleaning head can be e.g. a brush head and/or a flossing head. Thereby the reusable handle 600 can receive the code 200 stored within such a cleaning head by means of the data reader 102. Such a replaceable cleaning head may be available for the user in several different variants, each variant storing different code, which different code in turn causes the grant of access to different network-based service. When said code is being read out by the data reader 102 comprised in the oral care system, it is triggered that a sender of the oral care system starts to communicate with the external network outside the oral care system. The oral care system in this way can receive from and/or exchange data with said network, which allows offering the user network-based services, which he/she did not have in his oral care system, before said replaceable brush head was put together with said handle.

Furthermore, the reusable handle 600 comprises a user interface 110. The user interface 110 can be configured for receiving input and/or for outputting instructions to a user of the reusable handle 600, and is thus an interface integrated into a cleaning appliance of the oral care system 100. In other embodiments, the user interface of the oral care system may be embodied as e.g. a smart phone, a tablet, a computer, to which the handle can be connected.

As was described hereinbefore in detail the user interface 110 is configured for using the network-based service to which the oral care system gets access to when having read the code and having sent the signal to the network. Moreover, the oral care system is configured to change a functionality of the user interface 110 by carrying out the software reconfiguration. For example, the oral care system is configured, after having granted access to the network-based service, to provide to the user a diagnostic functionality regarding oral health of the user via the user interface 110.

Figure 3 shows a support unit 500, which is embodied as a reusable mouthpiece 700. The reusable mouthpiece 700 can be configured for being coupled to a replaceable cleaning part or cleaning head, e.g. comprising bristles. Furthermore, the reusable mouthpiece 700 can be configured for soft tissue treatment. Moreover, the reusable mouthpiece 700 can comprise the data reader 102 and the user interface 110 as was described hereinbefore for the handle 600 shown in figure 2.

Figure 4 shows a support unit 500 of an oral care system according to an exemplary embodiment of the invention, in which the support unit 500 is embodied as a reusable handle 600 of the oral care system. A replaceable cleaning head 610 can be attached to the reusable handle 600 and is embodied as a brush head. The brush head comprises the code 200 and/or a data storage, which stores said code 200. Furthermore, the reusable handle 600 comprises the data reader 102, which can receive the code 200 when the brush head 610 is attached and/or adjacent to the reusable handle 600. Also in this embodiment the user interface 110 is comprised by the reusable handle 600. In the embodiment of figure 4 the oral care system, comprising the handle and the cleaning head, is configured for uploading oral health data of the user to the network (see e.g. network 300 in figure 1). The diagnostic functionality provided by the oral care system, i.e. the network based service to which the handle and the cleaning head get access to, is based on the oral health data uploaded to the network. The oral care system shown in figure 4 is further configured for uploading, as health data of the user, data based on at least one image of an oral cavity of the user, which was captured by a camera (not shown here) that can be installed on the brush head.

Figure 5 shows another oral care system according to an exemplary embodiment of the present invention comprising a support unit 500, which is embodied as a reusable mouthpiece 700. The reusable mouthpiece 700 can be connected and/or attached to a replaceable cleaning part, which is embodied as an insertable mouthpiece 710 for brushing teeth, flossing teeth and/or soft tissue treatment, and which is also a component of the oral care system. The reusable mouthpiece 700 is configured for powering the insertable mouthpiece 710. Further, the insertable mouthpiece 710 comprises the code 200 and/or a data storage, which stores the code 200. Moreover, the reusable mouthpiece comprises a data reader for receiving the code from the insertable mouthpiece 710. The reusable mouthpiece 700 can comprise a user interface 110.

Figure 6 shows a flowchart illustrating the steps of the method 800 of granting access for an oral care system to a network-based service. The method comprises the step S1 of reading, by a data reader of an oral care system, a code comprised in a code-carrying element. Moreover, in step S2 a signal is generated based on the code read by the data reader and is sent by the oral care system to a receiver of a network outside the oral care system. Moreover, in step S3 access is granted to a network-based service in response to sending the signal to the receiver of the network. The method 800 comprise also the optional steps S4 and S5. In step S4 usage data indicative for a use of the oral care appliance are uploaded to the network, and the oral care system is configured to receive and process usage feedback data from the network as network-based service. In step S5 data relating to a diagnosis and/or usage feedback are presented to the user. This method allows for adding further functionalities to the oral care system, like e.g. analysis functionalities about oral care data from the system, which can be performed now on the oral care system and/or the network. This may lead to an improved oral care behavior of the user, and/or longer operating time of the oral care system because less computing power is needed within the oral care system.

Figure 7 shows a flowchart illustrating the method 810 of granting access for the oral care system 100 to a network-based service as an embodiment of the present invention. The method comprises the steps S1 to S5 as described before and hereinafter in view of the method 800. The method 810 further comprises the step S6 of uploading oral care data generated by the oral care system 100 to the network 300 by means of the data exchange 108. Moreover, the method 810 comprises the step of causing S7 a remote diagnosis procedure between the oral care system 100, in particular between a user interface 110 of the oral care system 100, and the network 300 by means of the data exchange 108. The step S8 of uploading individual use data of the oral care system 100 to the network 300 is also comprised by the method 810. Furthermore, the method 810 comprises the step S9 of downloading from the network 300 individual user instructions that are generated by the network 300 based on the uploaded individual use data.

Figure 8 shows a flowchart illustrating the steps of the method 900 for operating a network 300 according to an embodiment of the present invention. The method 900 comprises the step S10 of receiving a signal from the oral care system 100 by the network 300. The step S11 of selecting in the network 300 at least one oral care functionality to which access is granted for the oral care system 100 based on the received signal 104 can be comprised by the method 900. Furthermore, the method 900 comprises the step S12 of generating a server signal 106 based on the selected oral care functionality, wherein the server signal 106 is configured for initiating a software reconfiguration on the oral care system 100, as was described hereinbefore in detail. Further, the step S13 of sending the generated server signal to the oral care system 100 thereby initiating the software reconfiguration of the software installed on the oral care system 100 is comprised by the method 900.

Figure 9 shows a flowchart illustrating the steps of the method 910 for operating a network 300. The method 910 comprises the steps S10 to S13 as described before in the context of figure 8. The method 910 further comprises the step S14 of establishing a data connection between an external database and the network 300 based on the received signal. The step S15 of receiving, by the network 300, oral care data generated by the oral care system 100 is comprised by the method 910. Furthermore, the method 910 comprises the step S16 of exchanging the oral care data received from the oral care system 100 between the database and the network 300 via the established data connection.

Figure 10 shows an oral care system according to another embodiment of the present invention. The oral care system 100 comprises a support unit 500 embodied as a reusable handle 600. The oral care system 100 further comprises a brush head 610, which comprises a data storage storing the code 200. Furthermore, the oral care system 100 comprises a tablet 620 or alternatively a smartphone, which comprises a user interface 110 of the oral care system 100. Bluetooth or WiFi or the like can be used to connect the tablet 620 and the handle 600. The handle 600 further comprises a data reader 102. Before the data reader 102 receives the code 200, the user interface 110 comprises only a first functionality 630, which can be e.g. a timer for timing an oral care appliance. After the brush head 610 is attached to the handle 600 and the code 200 is received by the data reader 102, the data exchange (as was exemplarily described in detail before in the context of figure 1) can be established in a first step 622. During this first step 622 a second functionality 640 of the shown oral care system can be activated. For example, the second functionality 640 is the configuration for generating and uploading oral care data to the network 300 by means of the data exchange. Furthermore, based on the evaluation/analysis of the generated and uploaded data by means of the network 300 a remote diagnosis procedure can be triggered in a second step 624. The second step 624 for example can establish a third functionality 650 on the user interface 110 of the oral care system, which can be for example the ability to provide and use a video connection 122 between the user interface 110 of the oral care system 100 and a communication device of a dental professional such that the dental professional can support the user of the oral care system via the third functionality 650, i.e. the video connection 122.

One advantage of this oral care system and the corresponding method is that by replacing e.g. a cleaning head a functionality of the oral care system and/or the network can be unlocked or added. This can lead to new business models, which can generate added value for a user of the oral care system. A further advantage of this oral care system and the corresponding method is that with the help of this oral care system/the method further functionalities and/or analysis can be performed on the oral care system and/or the network, which can lead to an improved oral care behavior of the user, and/or longer operating time of the oral care system because less computing power is needed within the oral care system.

Where an indefinite or definite article is used when referring to a singular noun, e.g. "a", "an" or "the", this includes a plurality of that noun unless something else is specifically stated. It is to be understood that the terms so used are interchangeable under appropriate circumstances and that the embodiments of the invention described herein are capable of operation in other sequences than described or illustrated herein.

### LIST OF REFERENCE SIGNS:

- 100 -: oral care system
- 102 -: data reader
- 104 -: signal
- 106 -: server signal
- 108 -: data exchange
- 110 -: user interface
- 112 -: camera
- 120 -: communication device
- 122 -: video connection
- 200 -: code
- 300 -: network
- 500 -: support unit
- 600 -: reusable handle
- 610 -: brush head
- 620 -: tablet
- 622 -: first step
- 624 -: second step
- 630 -: first functionality
- 640 -: second functionality
- 650 -: third functionality
- 700 -: reusable mouthpiece
- 710 -: insertable mouthpiece
- 800 -: method of granting access for an oral care system to a network-based service
- 810 -: method of granting access for an oral care system to a network-based service
- 900 -: method for operating the computer network structure
- 910 -: method for operating the computer network structure

## Claims

1. An oral care system (100) for granting access to a network-based service, the oral care system comprising:
a data reader (102) for reading a code (200) comprised in a code-carrying element,
wherein the oral care system (100) is configured to send a signal (104) based on the code (200) read by the data reader (102) to a receiver of a network (300) outside the oral care system (100), and
wherein the oral care system (100) is configured for granting access to the network-based service in response to sending the signal to the receiver of the network (300).

2. An oral care system (100) according to claim 1,
wherein the oral care system is configured for reconfiguring software comprised in the oral care system (100) thereby granting the access to the network-based service, and
wherein the software reconfiguration carried out by the oral care system comprises unlocking software installed on the oral care system (100), installing new software on the oral care system (100), and/or changing software installed on the oral care system.

3. An oral care system (100) according to claim 1 or 2,
wherein the oral care system (100) comprises a user interface configured for using the network-based service, and
wherein the user interface is embodied as a smart phone, a tablet, a computer, or as an interface integrated into a cleaning appliance of the oral care system (100).

4. An oral care system (100) according to claims 2 and 3,
wherein the oral care system (100) is configured to change a functionality of the user interface by carrying out the software reconfiguration.

5. An oral care system (100) according to any of the preceding claims,
wherein the oral care system is configured, after having granted access to the network-based service, to provide to the user a diagnostic functionality regarding oral health of the user or an insurance functionality.

6. An oral care system according to claim 5,
wherein the oral care system (100) is configured for uploading oral health data of the user of the oral care system to the network (300), and
wherein the diagnostic functionality provided by the oral care system (100) is based on the oral health data uploaded to the network.

7. An oral care system (100) according to any of the preceding claims,
wherein the oral care system is configured for uploading, as health data of the user, data based on at least one image of an oral cavity of the user.

8. An oral care system (100) according to any of claims 5 to 7,
wherein the oral care system (100) is configured, in order to provide the diagnostic functionality, to
- receive from the network (300) data about a second opinion regarding the oral health of the user,
- process said received data about the second opinion, and to
- share the data about the second opinion with the user via a user interface of the oral care system.

9. An oral care system (100) according to any of the preceding claims,
wherein the oral care system (100) comprises an oral care appliance,
wherein the oral care system (100) is configured to upload to the network (300) usage data indicative for a use of the oral care appliance, and
wherein the oral care system (100) is configured to receive and process usage feedback data from the network as network-based service.

10. A method of granting access for an oral care system (100) to a network-based service, the method comprising
reading, by a data reader (102) of an oral care system (100), a code (200) comprised in a code-carrying element (S1),
sending a signal, based on the code (200) read by the data reader (102), by the oral care system (100) to a receiver of a network (300) outside the oral care system (S2), and
granting access to a network-based service in response to sending the signal to the receiver of the network (S3).

11. A method according to claim 10, further comprising
presenting data relating to a diagnosis and/or usage feedback to the user (S5).

12. A computer program, which instructs, when executed, a controller to control a system to perform a method of granting access for an oral care system (100) to a network-based service, the method comprising
reading, by a data reader (102) of an oral care system (100), a code (200) comprised in a code-carrying element,
sending a signal, based on the code (200) read by the data reader (102), by the oral care system (100) to a receiver of a network (300) outside the oral care system (100), and
granting access to a network-based service in response to sending the signal to the receiver of the network (300).

13. A controller comprising a computer program according to claim 12.

14. A code-carrying element comprising a code (200) and configured to facilitate a read out of the comprised code by a data reader of an oral care system (100), and
wherein the comprised code (200) is configured to allow granting access to a network-based service when sending a signal based on the code to a receiver of a network (300) outside the oral care system.

15. A code-carrying element according to claim 14,
wherein the code-carrying element is embodied as
- a cleaning head to be received by a handle of an oral care system, wherein the cleaning head is a brush head and/or a flossing head,
- a handle (600) of an oral care system for receiving a cleaning head being a brush head and/or a flossing head,
- a mouthpiece (700) of an oral care system configured for brushing teeth, flossing teeth and/or soft tissue treatment,
- a scratch card, and/or,
- a packaging for a cleaning head of an oral care system.
